# EUROPEAN PATENT SPECIFICATION

(11) **EP 1 638 527 B1**
(45) Date of publication and mention of the grant of the patent: **25.10.2006**
(21) Application number: 03741075.0
(22) Date of filing: 27.06.2003
(51) Int. Cl.: A61K 9/14

(54) **COMPOSITE PRODUCT OBTAINABLE BY COGRINDING OF AN ACTIVE PRINCIPLE WITH A COPOLYMER N-VINYL-2-PYRROLIDONE/VINYL-ACETATE**
ZUSAMMENSETZUNG, DURCH ZUSAMMEN VERMAHLEN EINES WIRKSTOFFES MIT EINEM N-VINYL-2-PYRROLIDON/VINYLACETATKOPOLYMERS ERHÄLTLICH
PRODUIT COMPOSITE OBTENU PAR BROYAGE D'UN PRINCIPE ACTIF AVEC UN COPOLYMERE N-VINYLE-2-PYRROLIDONE/VINYLE-ACETATE

(43) Date of publication of application: 29.03.2006
(73) Proprietor: BIOPROGRESS S.p.A., I-00165 Rome (IT)
(72) Inventor: OLIVIERI, Aldo, I-00165 Roma (IT); BONANOMI, Michele, I-00198 Roma (IT); PAZZI, Piergiorgio, I-66050 San Salvo (IT)
(74) Representative: Tansini, Elio Fabrizio
(86) International application number: PCT/IT2003/000401
(87) International publication number: WO 2005/000273

(56) References cited:
- WO-A-01/68100
- DE-A- 19 929 361
- GB-A- 1 560 406
- US-A- 5 741 519
- US-B1- 6 322 816

## Description

### FIELD OF THE INVENTION

The present invention relates to a composite product obtained by co-grinding of an active principle with a carrier comprising a N-vinylpyrrolidone/vinyl acetate copolymer. The present invention further relates to the use of said composite product for preparing pharmaceutical compositions aiming at improving absorption properties (shorter times for appearance of haematic peak, higher bioavailability) of drugs or active substances scarcely soluble in water environment.

### STATE OF THE ART

The improvement of absorption properties, i.e. of times for appearance of haematic peak and of bioavailability as the area under the haematic curve, of drugs or pharmaceutical active substances that are scarcely soluble in water environment, has been the object of several studies and quite different technical suggestions: micronization of the active substance with -subsequent reduction of granulometry and increase of surface area; formulation with surfactants; complexation with cyclodextrins and derivates; co-precipitation or extrusion with linear polymers.

Among the most innovative techniques that can enable to obtain products with improved biopharmaceutical properties, co-grinding of the scarcely soluble active substance with hydrophilic carriers has been among the most applied solutions thanks to its interesting results.

The use of co-grinding of scarcely soluble drugs with the water soluble linear polymer polyvinylpyrrolidone was described in 1975 (*Chem. Pharm. Bull.,* 23, 2973, 1975). In later publications (*Chem. Pharm. Bull.,* 78, 3340, 1977; *Chem. Pharm. Bull.,* 28, 652, 1980) microcrystalline cellulose was used as carrier for co-grinding.

The use of beta-cyclodextrin was described in JP Patent 7986607 and in DE Patent 3427788, in which lactose, calcium phosphate and starch are mentioned as further materials for co-grinding, to be added to cyclodextrin, if necessary.

Hydrophilic silica gel and other adsorbing inorganic materials were described in EP 129893, in which the obtained co-ground products are characterized by amorphizations of active substances and improved dissolution properties.

US Patent 4,639,370 describes co-grinding of scarcely soluble drugs with reticulated polymers that are insoluble but can be swelled in water, such as reticulated polyvinylpyrrolidone or reticulated sodium carboxymethyl cellulose: evident improvements of the properties concerning passage into solution and of absorption properties are obtained for very scarcely water soluble drugs such as methyl hydroxy progesterone acetate.

WO 9632931 describes the use of starch sodium glycolate as carrier with improvement of dissolution speed and anticipation of time of appearance of ibuprofen haematic peak.

In all documents referred to above co-grinding is carried out, leaving aside the kind of grinding mill or carrier used, on dry mixtures of the active substance, the carrier being introduced into the grinding chamber of the selected device.

In US Patent 5,354,560 and US Patent 5,449,521 the mixture of powders to be co-ground are introduced into grinding chambers that have been pre-saturated with water vapors or vapors from solvents that can solubilize the active substance. Here lower co-grinding times for obtaining improved properties of scarcely soluble drugs are claimed.

The improvements made to co-grinding technique by the introduction of solvent vapors into the co-grinding chambers, however, are negatively counterbalanced by higher process costs and by greater problems involving residues in the final product and by higher possibilities of degradation of the active substance.

DE 19929361 A discloses how an active principle is incorporated into a PVP-VA melt (solid solution) and then coground to a powder/granulate. DE 19929361 A does not disclose just a cogrinding of both components in powder form.

WO 01/68100 A discloses torasemide preparations, in which the active principle is incorporated into a NVP-VA (N-vinylpyrrolidone-vinylacetate copolymer) melt and then coground to a powder/granulate. WO 01/68100 A does not disclose just a cogrinding of both components in powder form.

US 6322816 B discloses ibuprofen in a porous polymer matrix; as a suitable embodiment a copolymer NVP-VA is indicated. No cogrinding of both components in powder form is disclosed.

US 5741519 B discloses a solid solution of an active principle in a NVP-VA matrix. No cogrinding of both components in powder form is disclosed.

### DESCRIPTION OF THE INVENTION

It has now been surprisingly found that the use of linear copolymer N-vinyl-2-pyrrolidone/vinyl acetate as carrier for co-grinding of scarcely water soluble drugs results in improvements of the crystalline structure of said drugs (reduction of melting enthalpy and/or melting point), greater increase of solubility and of dissolution speed with respect to, co-grinding times being the same, what can be obtained by using other carriers commonly used for co-grinding, such as for instance linear polyvinylpyrrolidone, reticulated polyvinylpyrrolidone or cyclodextrins.

The technical features and the advantages deriving from the use of N-vinyl-2-pyrrolidone/vinyl acetate copolymer (hereinafter referred to as NVP/VA for reasons of shortness) as carrier for co-grinding of scarcely soluble drugs according to the present invention will be evident from the following detailed description.

In a preferred embodiment of the present invention the selected active substance and NVP/VA carrier, both in powder form, are pre-mixed in a suitable powder mixer.

Preferably, powder granulometry can vary within a range between 0.01 and 1,000 microns both for the carrier and for the drug; for instance it can vary between 0.1 and 200 microns.

Preferably, the mixture comprising the selected active substance and NVP/VA carrier is introduced into the grinding chamber of any grinding mill, together with the grinding means.

Alternatively the mixture comprising the selected active substance and NVP/VA carrier can be introduced directly into the grinding mill without pre-mixing.

The mill that can be used for co-grinding an active substance with NVP/VA carrier comprises a grinding chamber housing grinding means of any kind (for instance balls or cylinders).

Co-grinding comprises for instance a mechanical stirring carried out by rolling, centrifugal rotation or vibration.

Co-grinding can be carried out at low or high energy for times varying from 0.1 to 48 hours; preferably for times between 0.5 and 8 hours.

Preferably, the weight ratio of NVP/VA carrier to active substance can be between 200:1 and 1:10; still more preferably between 100:1 and 1:5; for instance between 10:1 and 0.5:1.

At the end of co-grinding the resulting powder comprising the composite product according to the present invention can be sieved or used directly in the preparation of the pharmaceutical composition in the desired pharmaceutical form, for instance tablet, capsule, packet, powder, pellet, syrup or solution.

The pharmaceutical composition comprising the composite product is prepared by means of the technique known to the person skilled in the art and by using excipients and pharmaceutically acceptable additives commonly used for preparing the desired pharmaceutical forms.

Many different classes of drugs can be usefully worked with the technique of the present invention, from anti-inflammatory agents to analgesics, relaxants, anti-microbic agents, antiseptics, acid pump inhibitors, H₂ antagonists, anti-emetics and anti-nausea, biliary acids, oral hypoglycemizers, diuretics, anti-hypertensives, sulfonamides, ace-inhibitors, hypolipemizers, anti-mycotic agents, antihistamines, hormones, quinolone derivates, antibacterial agents, beta-lactame and fluoroquinolone antibiotics, antiviral agents, anti-neoplastic agents, immuno-modulators and immuno-suppressors, anti-gout agents, anesthetics, analgesics, antipyretics, 5HT₁ agonists, anti-Parkinson agents, anti-psychotic agents, tranquillizers, antidepressants, anti-parasitic agents, non-cortisone anti-allergic agents, anti-asthmatic agents, anti-glaucoma agents, inhibitors of carbonic anhydrase, beta-blockers, and others.

Drugs to which the present invention can be applied, whatever the therapeutic class they belong to, are scarcely water soluble drugs and drugs with low dissolution speed.

Non-exhaustive examples are paracetamol, nifedipine, piroxicam, ibuprofen, sulindac, diclofenac, alclofenac, ketorolac, indomethacine, naproxen, fenoprofen, flurbiprofen, ketoprofen, cimetidine, ranitidine, mesalazine, ursodeoxycholic acid, mefenamic acid, sinvastatin, megestrol acetate, lorazepam, diazepam, cyclosporin, ubiquinone, tolbutamide, ketanserine, furosemide, nicergoline, losartan, econazole, miconazole, taxol, progesterone, prednisolone, beclometasone, nalidixic acid, finasteride, ciprofloxacine, ofloxacine, lomefloxacine, methotrexate, etoposide, daunorubicine, tamoxifen, allopurinol, clodronic acid, sumatriptan, carbamazepine, clorpromazine, clozapine, sulpiride, buspirone, fluoxetine, citalopram, caffeine, metronidazole, acetazolamide etc.

N-2-vinyl-pyrrolidone/vinyl acetate copolymer is a flowing powder with a high pharmaceutical workability thanks to the spray-drying process used to obtain it. Said spray-drying technique results in spherical particles with a limited and homogenous size distribution. Said morphology of the particles positively affects the flow of the powder and its ability to mix with other excipients.

Another very important property of said copolymer is its low glass transition temperature (Tg).

By mere comparison, Tg of NVP/VA is of about 106°C, whereas the one of the corresponding linear polymer N-vinylpyrrolidone is of about 160°C.

Glass transition temperature can be defined as the temperature at which a polymer starts to get fluid without being completely melted.

The Applicant has found it useful to exploit the technical property of Tg of NVP/VA for preparing pharmaceutical forms for instance as tablets. As a matter of fact, during the compression of the polymeric powders a low glass transition temperature helps the formation of inter-particle bonds thanks to an easier deformation/fluidization of the single particles. The formation of inter-particle bonds enables to obtain a tablet pharmaceutical form having a higher hardness than the one that could be obtained with other traditional excipients by compression, such as for instance lactose or microcrystalline cellulose.

Said better compression properties of NVP/VA copolymer have led the Applicant to use successfully said copolymer as carrier for co-grinding of little soluble drugs so as to obtain powders of drug/carrier composite with improved compression properties with respect to commonly used carriers such as linear polymers, for instance polyvinylpyrrolidone, cyclodextrins, reticulated polymers, for instance crospovidone.

Beyond better compression properties NVP/VA copolymer has a lower hygroscopicity with respect to the corresponding linear polymer polyvinylpyrrolidone.

This lower hygroscopicity involves a higher stability of the copolymer during its conservation (preservation of flow and compression properties) and a lower negative impact on the stability of moisture-degradable drugs mixed or better co-ground with said carrier.

Now, it has been unexpectedly found that beyond better compression properties of the resulting powders, co-ground products deriving from scarcely soluble drugs and NVP/VA have lower melting enthalpies and lower melting temperatures with respect to co-ground products obtained with previously used carriers such as polyvinylpyrrolidone, cyclodextrins, crospovidone.

The following examples of co-ground products obtained with NVP/VA (examples 1-8) are given to a mere illustrative and non-exhaustive purpose for the invention, compared with co-ground products obtained with other commonly used carriers (examples A-O).

The properties of co-ground products obtained are described in tables 1-7.

### EXAMPLES

1 - 16.6 g of nimesulide are mixed with 49.8 g of NVP/VA for 15'. The powder is then poured into the grinding chamber of a low energy vibrational mill. Grinding is carried out for 3 hours.
2 - 13.3 g of nimesulide are mixed with 53.2 g of NVP/VA for 15'. The powder is then poured into the grinding chamber of a low energy vibrational mill. Grinding is carried out for 3 hours.
3 - 16.6 g of nimesulide are mixed with 49.8 g of NVP/VA for 15'. The powder is then poured into the grinding chamber of a low energy vibrational mill. Grinding is carried out for 2 hours.
4 - 13.3 g of nimesulide are mixed with 53.2 g of NVP/VA for 15'. The powder is then poured into the grinding chamber of a low energy vibrational mill. Grinding is carried out for 2 hours.
5 - 2.5 g of piroxicam are mixed with 12.5 g of NVP/VA for 15'. The powder is then poured into the grinding chamber of a centrifugal mill. Grinding is carried out for 4 hours.
6 - 180.0 g of nifedipine are mixed with 800.0 g of NVP/VA for 15'. The powder is then poured into the grinding chamber of a high energy vibrational mill. Grinding is carried out for 4 hours.
7 - 13.3 g of ursodeoxycholic acid (UDCA) are mixed with 53.2 g of NVP/VA for 15'. The powder is then poured into the grinding chamber of a low energy vibrational mill. Grinding is carried out for 4 hours.
8 - 11.1 g of ursodeoxycholic acid (UDCA) are mixed with 55.5 g of NVP/VA for 15'. The powder is then poured into the grinding chamber of a low energy vibrational mill. Grinding is carried out for 4 hours.

A - 16.6 g of nimesulide are mixed with 49.8 g of PVP for 15'. The powder is then poured into the grinding chamber of a low energy vibrational mill. Grinding is carried out for 3 hours.
B - 13.3 g of nimesulide are mixed with 53.2 g of PVP for 15'. The powder is then poured into the grinding chamber of a low energy vibrational mill. Grinding is carried out for 3 hours.
C - 16.6 g of nimesulide are mixed with 49.8 g of PVP-CL for 15'. The powder is then poured into the grinding chamber of a low energy vibrational mill. Grinding is carried out for 3 hours.
D - 13.3 g of nimesulide are mixed with 53.2 g of PVP-CL for 15'. The powder is then poured into the grinding chamber of a low energy vibrational mill. Grinding is carried out for 3 hours.
E - 16.6 g of nimesulide are mixed with 49.8 g of PVP for 15'. The powder is then poured into the grinding chamber of a low energy vibrational mill. Grinding is carried out for 2 hours.
F - 13.3 g of nimesulide are mixed with 53.2 g of PVP for 15'. The powder is then poured into the grinding chamber of a low energy vibrational mill. Grinding is carried out for 2 hours.
G - 16.6 g of nimesulide are mixed with 49.8 g of PVP-CL for 15'. The powder is then poured into the grinding chamber of a low energy vibrational mill. Grinding is carried out for 2 hours.
H - 13.3 g of nimesulide are mixed with 53.2 g of PVP-CL for 15'. The powder is then poured into the grinding chamber of a low energy vibrational mill. Grinding goes on for 2 hours.
I - 2.5 g of piroxicam are mixed with 12.5 g of PVP-CL for 15'. The powder is then poured into the grinding chamber of a centrifugal mill. Grinding is carried out for 4 hours.
L - 2.5 g of piroxicam are mixed with 12.5 g of β-cyclodextrin for 15'. The powder is then poured into the grinding chamber of a centrifugal mill. Grinding is carried out for 4 hours.
M - 180.0 g of nifedipine are mixed with 900.0 g of PVP for 15'. The powder is then poured into the grinding chamber of a high energy vibrational mill. Grinding is carried out for 4 hours.
N - 13.3 g of UDCA are mixed with 53.2 g of β-cyclodextrin for 15'. The powder is then poured into the grinding chamber of a low energy vibrational mill. Grinding is carried out for 4 hours.
O - 11.1 g of UDCA are mixed with 55.5 g of β-cyclodextrin for 15'. The powder is then poured into the grinding chamber of a low energy vibrational mill. Grinding is carried out for 4 hours.

### LIST OF TABLES

Table 1: Variation of ΔH_{f} and of T_{f} of Nimesulide complex with different Carriers at 40°C and 75% of relative humidity (R.H.).
Table 2: Co-grinding test of Nimesulide with different Carriers.
Table 3: Co-grinding test of Piroxicam with various Carriers (weight ratio piroxicam/carrier: 1/5).
Table 4: Co-grinding test of Nifedipine with various Carriers (weight ratio nifedipine/carrier: 1/5).
Table 5: Co-grinding test of UDCA with various Carriers.
Table 6: Co-grinding test of UDCA with various Carriers (weight ratio nimesulide/carrier: 1/4). Percentage release is given.
Table 7: Dissolution speed of Piroxicam co-ground with different Carriers (weight ratio nimesulide/carrier: 1/4). Percentage release is give.

### CHARACTERIZATION TESTS

Powders of co-ground products obtained by using NVP/VA carrier have been characterized by:
a). using differential scanning calorimetry
b). measuring dissolution speed
and compared with co-ground products obtained with commonly used carriers.

Operating conditions of the tests were the following: Differential scanning calorimetry

A differential scanning calorimeter Perkin Elmer, mod. Pyris 1, with nitrogen flow, and a heating speed of 10°C/min has been used.

### Measurement of dissolution speed

The method referred to in USP XXI, no. 2 has been used, using SOTAX apparatus, with thermostatation of dissolution means at 37°C, and a rotation speed of the blades of 100 rpm. The concentration of dissolved drug has been measured by means of a spectrophotometer (Perkin Elmer, mod. Lambda 25).

In the case of piroxicam as dissolution mean HC1 O.1N, pH 1.2 has been used; in the case of nimesulide phosphate buffer pH 7.5.

Table 1 contains data referring to melting enthalpy and temperature of co-ground products obtained from nimesulide with the carrier according to the present invention NVP/VA and with comparative carriers linear and reticulated PVP, under different conditions and conservation times: it can be observed that NVP/VA has a clearly higher ability in de-structuring nimesulide (lower melting enthalpies and temperatures) and in keeping said activation under the various conservation conditions.

Table 2 contains calorimetry data referring to co-ground products obtained from nimesulide with different carriers, with different weight ratios carrier/drug and different co-grinding times: in all cases co-ground products with NVP/VA are more destructured.

Table 3 contains calorimetry data referring to co-ground products obtained from piroxicam with different carriers (NVP/VA, PVP-CL, beta-cyclodextrin): also in this case NVP/VA carrier gives rise to lower melting enthalpies and temperatures.

In Table 4 said higher de-structuring ability of NVP/VA is shown for co-ground products with nifedipine, whereas Table 5 shows data referring to co-ground products obtained from ursodeoxycholic acid, which here again are more activated with NVP/VA carrier.

Unexpectedly again, higher dissolution speeds for co-ground products with NVP/VA with respect to commonly used carriers have been found, as shown in Table 6 for nimesulide and Table 7 for piroxicam. Said better dissolution properties are in line with the better properties of chemical-physical activation.

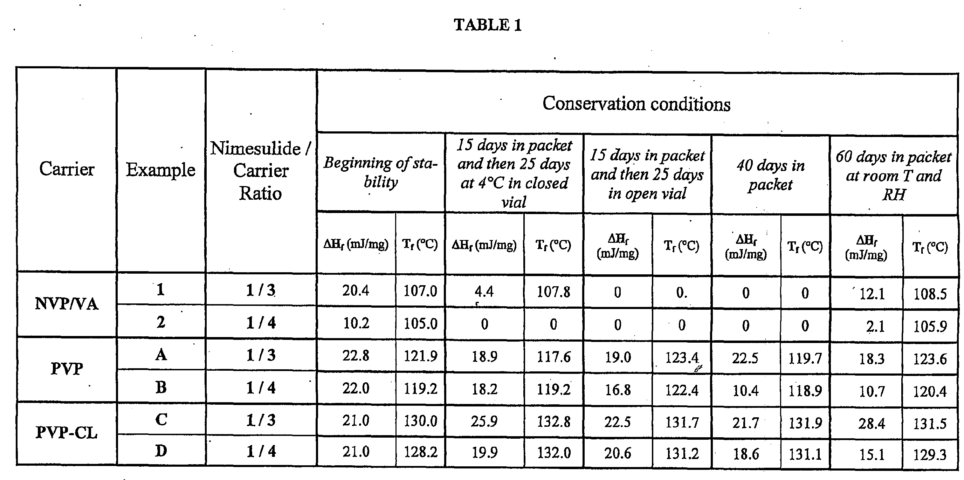

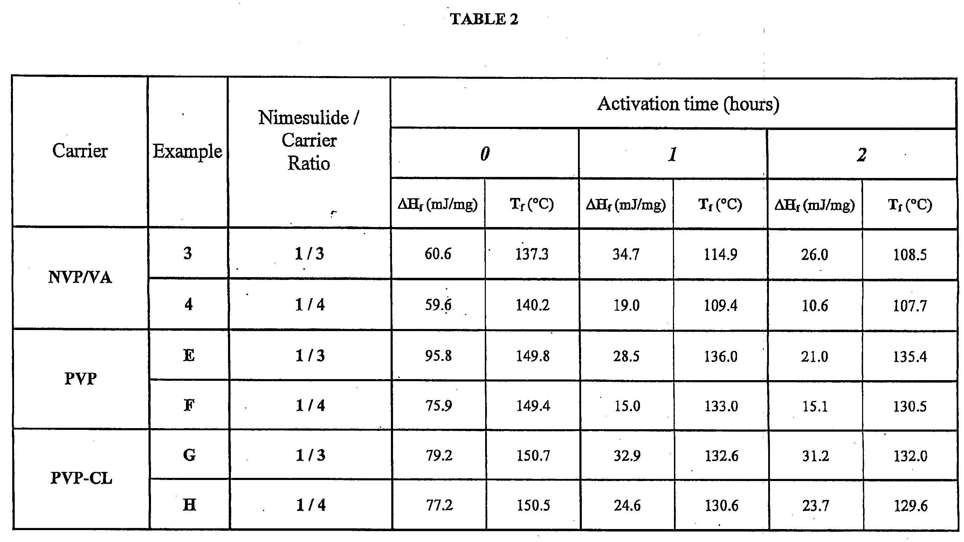

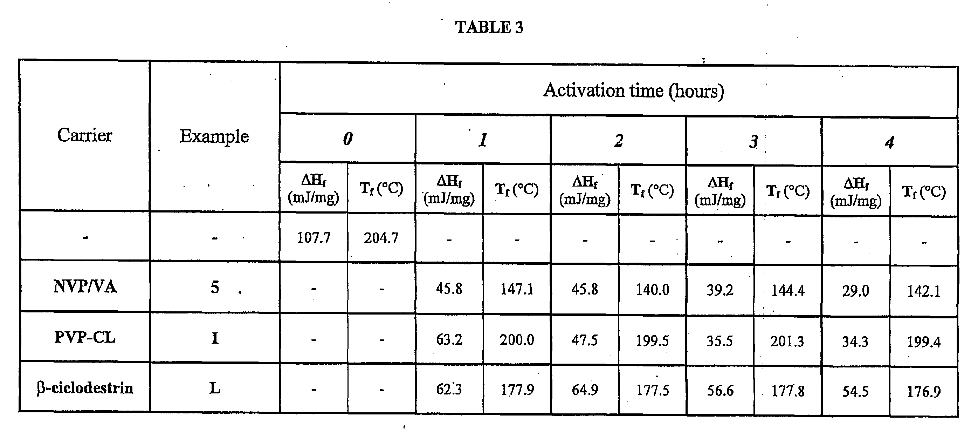

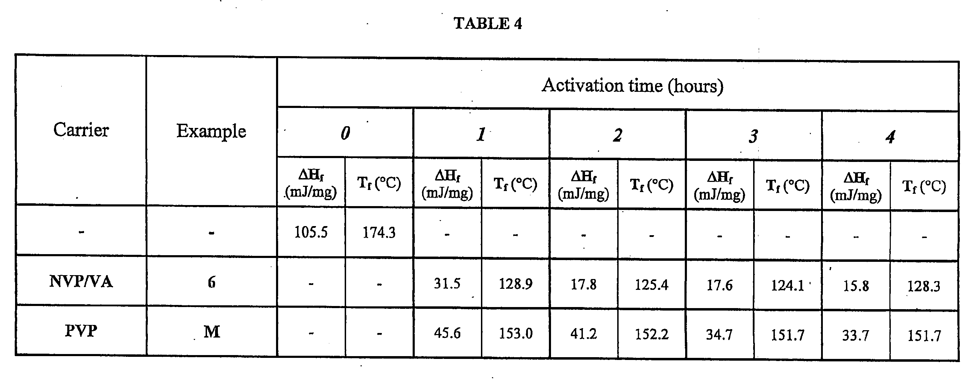

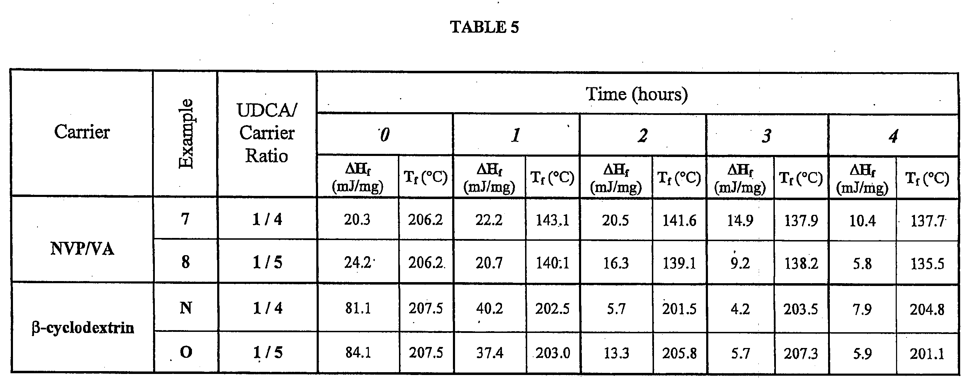

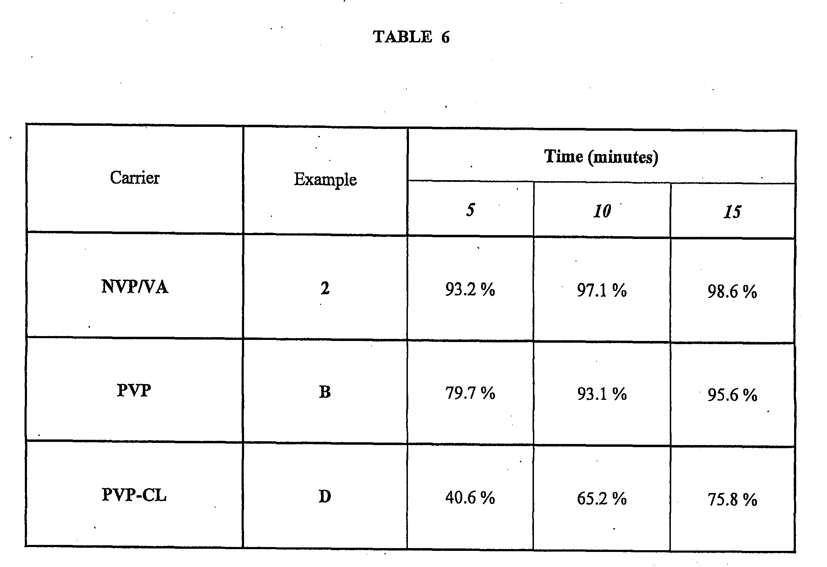

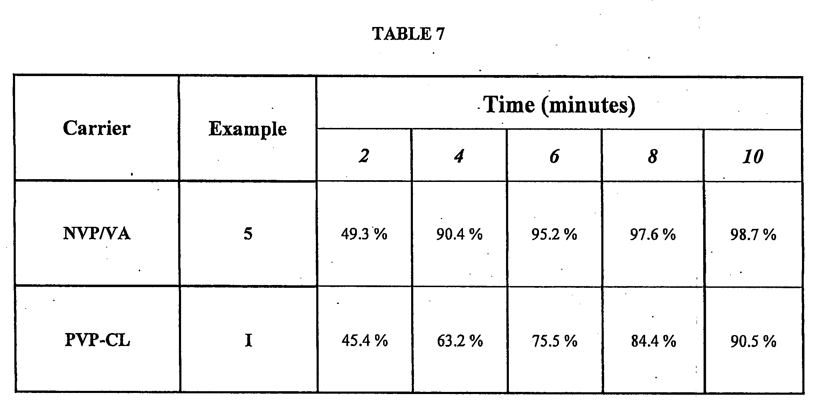

## Claims

1. Method for preparing a composite product comprising a step in which an active substance in powder form undergoes co-grinding with a carrier comprising N-vinyl-2-pyrrolidone/vinyl acetate copolymer in powder form.

2. Method according to claim 1, in which the carrier is N-vinyl-2-pyrrolidone/vinyl acetate.

3. Method according to claim 1, in which the co-grinding step takes place in dry conditions.

4. Method according to claim 1, in which the active substance is chosen among non steroidal anti-inflammatory agents.

5. Method according to claim 1, in which the active substance is chosen among anti-hypertensives.

6. Method according to claim 1, in which the active substance is chosen among hepato-biliary agents.

7. Method according to claim 1, in which the active substance is chosen among substances that are scarcely soluble in water environment.

8. Method according to claim 7, in which the active substance is chosen among scarcely water soluble substances having a low dissolution speed.

9. Method according to at least one of the preceding claims, in which the active substance is chosen among: anti-inflammatory agents, analgesics, relaxants, anti-microbic agents, antiseptics, acid pump inhibitors, H₂ antagonists, anti-emetics and anti-nausea, biliary acids, oral hypoglycemizers, diuretics, anti-hypertensives, sulfonamides, ace-inhibitors, hypolipemizers, anti-mycotic agents, antihistamines, hormones, quinolone derivates, antibacterial agents, beta-lactame and fluoroquinolone antibiotics, antiviral agents, anti-neoplastic agents, immuno-modulators and immuno-suppressors, anti-gout agents, anesthetics, analgesics, antipyretics, 5HT₁ agonists, anti-Parkinson agents, anti-psychotic agents, tranquillizers, antidepressants, anti-parasitic agents, non-cortisone anti-allergic agents, anti-asthmatic agents, anti-glaucoma agents, inhibitors of carbonic anhydrase or beta-blockers.

10. Method according to claim 9, in which the active substance is chosen among: paracetamol, nifedipine, piroxicam, ibuprofen, sulindac, diclofenac, alclofenac, ketorolac, indomethacine, naproxen, fenoprofen, flurbiprofen, ketoprofen, cimetidine, ranitidine, mesalazine, ursodeoxycholic acid, mefenamic acid, sinvastatin, megestrol acetate, lorazepam, diazepam, cyclosporin, ubiquinone, tolbutamide, ketanserine, furosemide, nicergoline, losartan, econazole, miconazole, taxol, progesterone, prednisolone, beclometasone, nalidixic acid, finasteride, ciprofloxacine, ofloxacine, lomefloxacine, methotrexate, etoposide, daunorubicine, tamoxifen, allopurinol, clodronic acid, sumatriptan, carbamazepine, clorpromazine, clozapine, sulpiride, buspirone, fluoxetine, citalopram, caffeine, metronidazole, acetazolamide.

11. Method according to at least one of the preceding claims, in which the active substance and N-vinyl-2-pyrrolidone/vinyl acetate copolymer are present in a weight ratio between 1:200 and 10:1; preferably between 1:100 and 5:1.

12. Method according to at least one of the preceding claims, in which the active substance and N-vinyl-2-pyrrolidone/vinyl acetate copolymer are premixed in powder mixer.

13. Method according to at least one of the preceding claims, in which the mixture comprising the active substance and N-vinyl-2-pyrrolidone/vinyl acetate copolymer is introduced into the grinding mill without premixing.

14. Method according to at least one of the preceding claims, in which powder granulometry of both the active substance and N-vinyl-2-pyrrolidone/vinyl acetate copolymer is within a range between 0.01 and 1,000 microns.

15. Method according to at least one of the preceding claims, in which co-grinding is carried out at low or high energy for times varying from 0.1 to 48 hours; preferably for times between 0.5 to 8 hours.

## Patentansprüche

1. Verfahren zur Herstellung einer Zusammensetzung umfassend einen Schritt, bei dem ein pulverförmiger Wirkstoff einer Zusammenvermahlung mit einem Träger umfassend ein pulverförmiges Copolymer N-Vinyl-2-pyrrolidon/Vinylacetat unterworfen wird.

2. Verfahren nach Anspruch 1, worin der Träger N-Vinyl-2-pyrrolidon/Vinylacetat ist.

3. Verfahren nach Anspruch 1, worin der Zusammenvermahlungsschritt unter trockenen Bedingungen stattfindet.

4. Verfahren nach Anspruch 1, worin der Wirkstoff aus nichtsteroidalen Antiphlogistika ausgewählt wird.

5. Verfahren nach Anspruch 1, worin der Wirkstoff aus Antihypertonika ausgewählt wird.

6. Verfahren nach Anspruch 1, worin der Wirkstoff aus Leber-Gallenmitteln ausgewählt wird.

7. Verfahren nach Anspruch 1, worin der Wirkstoff aus Stoffen, die in einer wässrigen Umgebung kaum löslich sind, ausgewählt wird.

8. Verfahren nach Anspruch 7, worin der Wirkstoff aus kaum wasserlöslichen Stoffen mit einer geringen Lösungsgeschwindigkeit ausgewählt wird.

9. Verfahren nach mindestens einem der vorherigen Ansprüche, worin der Wirkstoff aus: Antiphlogistika, Analgetika, Antispastika, antimicrobielle Mitteln, Antiseptika, Säurepumpenhemmern, H₂-Antagonisten, Antibrechmittel und Antiemetika, Gallensäuren, oralen Hypoglykämika, Diuretika, Antihypertonika, Sulfonamiden, ACE-Hemmern, Hypolipämie-Mitteln, Antipilzmitteln, Antihistaminika, Hormonen, Quinolon-Derivaten, Antibakterika, Beta-Lactam- und Fluoroquinolon-Antibiotika, Antiviralen, Antineoplastika, Immunomodulatoren und Immunosuppressoren, Antigichtmitteln, Anästhetika, Analgetika, Antipyretika, 5HT₁-Agonisten, Antiparkisonmitteln, Antipsychotika, Beruhigungsmitteln, Antidepressiva, Antiparasitika, Nicht-Kortison-Antiallergika, Antiasthmatika, Antiglaukommiteln, Carbonanhydrase-Hemmern oder Beta-Blockern, ausgewählt wird.

10. Verfahren nach Anspruch 9, worin der Wirkstoff aus: Paracetamol, Nifedipin, Piroxicam, Ibuprofen, Sulindac, Diclofenac, Alclofenac, Ketorolac, Indomethazin, Naproxen, Fenoprofen, Flurbiprofen, Ketoprofen, Cimetidin, Ranitidin, Mesalazin, Ursodeoxychlonsäure, Mefenaminsäure, Sinvastatin, Megestrolacetat, Lorazepam, Diazepam, Cyclosporin, Ubiquinon, Tolbutamid, Ketanserin, Furosemid, Nicergolin, Losartan, Econazol, Miconazol, Taxol, Progesteron, Prednisolon, Beclomethason, Nalixidinsäure, Finasterid, Ciprofloxacin, Ofloxacin, Lomefloxacin, Methotrexat, Etoposid, Daunorubicin, Tamoxifen, Allopurinol, Clodronsäure, Sumatriptan, Carbamazepin, Clorpromazin, Clozapin, Sulpirid, Buspiron, Fluoxetin, Citalopram, Koffein, Metronidazol, Acetazolamid, ausgewählt wird.

11. Verfahren nach mindestens einem der vorherigen Ansprüche, worin der Wirkstoff und das Copolymer N-Vinyl-2-pyrrolidon/Vinylacetat bei einem Gewichtverhältnis von 1:200 bis 10:1, bevorzugt von 1:100 bis 5:1, vorliegen.

12. Verfahren nach mindestens einem der vorherigen Ansprüche, worin der Wirkstoff und das Copolymer N-Vinyl-2-pyrrolidon/Vinylacetat in einem Pulvermischer vorgemischt werden.

13. Verfahren nach mindestens einem der vorherigen Ansprüche, worin das Gemisch umfassend den Wirkstoff und das Copolymer N-Vinyl-2-pyrrolidon/Vinylacetat in die Mahlmühle ohne Vormischen eingelegt wird.

14. Verfahren nach mindestens einem der vorherigen Ansprüche, worin die Pulvergranulometrie des Wirkstoffs und des Copolymers N-Vinyl-2-pyrrolidon/Vinylacetat in einem Bereich von 0,01 und 1.000 Mikron liegt.

15. Verfahren nach mindestens einem der vorherigen Ansprüche, worin die Zusammenvermahlung bei geringer oder hoher Energie für Zeiten von 0,1 bis 48 Stunden, bevorzugt für Zeiten von 0,5 bis 8 Stunden, durchgeführt wird.

## Revendications

1. Méthode pour la préparation d'un produit composite comprenant une étape dans laquelle une substance activé en forme de poudre est soumise à un co-broyage avec un support comprenant un copolymère N-vinyl-2-pyrrolidone/vinylacétate en forme de poudre.

2. Méthode selon la revendication 1, où le support est N-vinyl-2-pyrrolidone/vinylacétate.

3. Méthode selon la revendication 1, où l'étape de co-broyage a lieu à sec.

4. Méthode selon la revendication 1, où la substance active est choisie parmi les anti-inflammatoires non stéroïdiens.

5. Méthode selon la revendication 1, où la substance active est choisie parmi les anti-hypertensives.

6. Méthode selon la revendication 1, où la substance active est choisie parmi les agents hépato-biliaires.

7. Méthode selon la revendication 1, où la substance active est choisie parmi des substances étant peu solubles dans un environnement aqueux.

8. Méthode selon la revendication 7, où la substance active est choisie parmi des substance peu hydrosolubles ayant une vitesse de dissolution basse.

9. Méthode selon au moins une des revendications précédentes, où la substance active est choisie parmi: anti-inflammatoires, analgésiques, antispasmodiques, agents antimicrobiens, antiseptiques, inhibiteurs de la pompe acide, antagonistes de H₂, agents antiémétiques et antinausée, acides biliaires, agents hypoglycémiants oraux, diurétiques, anti-hypertensifs, sulfonamides, ACE-inhibiteurs, agents hypolipémiants, antimycosiques, antihistaminiques, hormones, dérivés de quinolone, agents antibactériens, antibiotiques à bêta-lactam et fluoroquinolone, antiviraux, agents anti-néoplastiques, immunomodulateurs et immunosuppresseurs, agents anti-goutte, anesthétiques, analgésiques, antipyrétiques, agonistes de 5HT₁, agents anti-Parkinson, agents anti-psychotiques, tranquillisants, antidépresseurs, antiparasitaires, antiallergiques non cortisoniques, antiasthmatiques, agents anti-glaucome, inhibiteurs de l'anhydrase carboniques ou bêtabloquants.

10. Méthode selon la revendication 9, où la substance active est choisie parmi: paracétamol, nifédipine, piroxicam, ibuprofène, sulindac, diclofénac, alclofénac, kétorolac, indométhacine, naproxène, fénoprofène, flurbiprofène, kétoprofène, cimétidine, ranitidine, mésalazine, acide ursodéoxycholique, acide méfénamique, sinvastatine, acétate de mégestrol, lorazépam, diazépam, cyclosporine, ubiquinone, tolbutamide, kétansérine, furosémide, nicergoline, losartan, éconazole, miconazole, taxol, progestérone, prednisolone, béclométasone, acide nalidixique, finastéride, ciprofloxacine, ofloxacine, loméfloxacine, méthotrexate, étoposide, daunorubicine, tamoxifène, allopurinol, acide clodronique, sumatriptan, carbamazépine, clopromazine, clozapine, sulpiride, buspirone, fluoxétine, citalopram, caféine, métronidazole, acétazolamide.

11. Méthode selon au moins une des revendications précédentes, où la substance active et le copolymère N-vinyl-2-pyrrolidone/vinylacétate sont présents dans un rapport en poids compris entre 1:200 et 10:1, de préférence entre 1:100 et 5:1.

12. Méthode selon au moins une des revendications précédentes, où la substance active et le copolymère N-vinyl-2-pyrrolidone/vinylacétate sont pré-mélangés dans un mélangeur de poudres.

13. Méthode selon au moins une des revendications précédentes, où le mélange comprenant la substance active et le copolymère N-vinyl-2-pyrrolidone/vinylacétate est introduit dans le broyeur sans être pré-mélangé.

14. Méthode selon au moins une des revendications précédentes, où la granulométrie des poudres de la substance active et du copolymère N-vinyl-2-pyrrolidone/vinylacétate est comprise dans un intervalle de 0,01 à 1.000 microns.

15. Méthode selon au moins une des revendications précédentes, où le co-broyage est effectué à basse ou haute énergie pour des périodes de 0,1 à 48 heures, de préférence pour des périodes de 0,5 à 8 heures.
